# EUROPÄISCHE PATENTSCHRIFT

(11) **EP 0 888 275 B1**
(45) Veröffentlichungstag und Bekanntmachung des Hinweises auf die Patenterteilung: **16.05.2001**
(21) Anmeldenummer: 97908194.0
(22) Anmeldetag: 07.03.1997
(51) Int. Cl.: C07C 45/77, C07C 49/92, C07C 67/00, C07C 69/72

(54) **VERFAHREN ZUR HERSTELLUNG VON ERDALKALIMETALLSALZEN ALIPHATISCHER BETA-KETOVERBINDUNGEN**
METHOD OF PRODUCING ALKALINE-EARTH SALTS OF ALIPHATIC BETA-KETO COMPOUNDS
PROCEDE DE PRODUCTION DE SELS D'ALCALINOTERREUX DE COMPOSES BETA-CETO ALIPHATIQUES

(30) Priorität: 15.03.1996 DE 19610320
(43) Veröffentlichungstag der Anmeldung: 07.01.1999
(73) Patentinhaber: Cognis Deutschland GmbH, 40589 Düsseldorf (DE)
(72) Erfinder: WOLLMANN, Gerhard, D-40723 Hilden (DE); KLAMANN, Jörg-Dieter, D-27574 Bremerhaven (DE); SONNEN, Guido, D-47443 Moers (DE)
(86) Internationale Anmeldenummer: EP9701156
(87) Internationale Veröffentlichungsnummer: WO9734859

(56) Entgegenhaltungen:
- EP-A- 0 514 893
- WO-A-91/13051
- FR-A- 1 576 711
- CHEMICAL ABSTRACTS, vol. 088, no. 19, 8.Mai 1978 Columbus, Ohio, US; abstract no. 136140, OHHARA M ET AL: "Alkaline earth metal salts of aliphatic.beta.-keto compounds" XP002032636 in der Anmeldung erwähnt & JP 52 136 131 - (OHARA YAKUHIN KOGYO CO., LTD.;JAPAN) 14.November 1977

## Beschreibung

Die Erfindung betrifft ein Verfahren zur Herstellung von Erdalkalimetallsalzen aliphatischer β-Ketoverbindungen durch die Umsetzung aliphatischer β-Ketoester oder β-Diketone mit einem Erdalkalimetallhydroxid in Abwesenheit eines Lösungsmittels zu den entsprechenden Erdalkalimetallsalzen, dabei wird die Reaktion derart durchgeführt, daß das pulverförmige Erdalkalimetallhydroxid vorgelegt und portionsweise die β-Ketoverbindung zudosiert wird.

### Stand der Technik

Erdalkalimetallsalze aliphatischer β-Ketoverbindungen wie z. B. Calciumsalze von Ethylacetoacetat und Acetylaceton werden üblicherweise als nichttoxische Stabilisatoren für PVC eingesetzt und substituieren damit die toxischen Bleiverbindungen. Aus **Z. h. Obsche Khim 42 403 (1972)** ist ein Verfahren zur Herstellung eines Calciumsalzes von Ethylacetoacetat durch Behandlung von Ethylacetoacetat mit metallischem Calcium in absolutem Ethanol bekannt. Dieses Verfahren ist bedingt durch den Einsatz metallischen Calciums sehr aufwendig und teuer. In **J. Chem. Soc., 1951, 2505** wird ein weiteres Verfahren beschrieben, bei dem Ethylacetoacetat mit Calciumcarbonat oder Calciumoxid in absolutem Benzol behandelt wird. Die Umsetzung mit Calciumoxid ist durch eine sehr geringe Reaktionsgeschwindigkeit gekennzeichnet, während bei der Verwendung von Calciumcarbonat höhere Kosten entstehen und die Dosierung problematisch ist. In beiden Fällen treten während der Reaktion extrem hohe Viskositäten auf und die Farbgebung der Produkte ist zu dunkel. Weiterhin ist aus der **DE 1,039,056** ein Verfahren zur Synthese von Erdalkalimetalisalzen von Acetylaceton bekannt, bei dem Acetylaceton mit Ammoniak behandelt und das gebildete Ketimin anschließend mit einem Metallsalz umgesetzt wird. In diesem Fall wird durch die Verwendung von Calciumhydroxid zwar eine höhere Reaktionsgeschwindigkeit erreicht, jedoch treten weiterhin hohe Viskositäten im Verlauf der Reaktion auf. Die Farbgebung der Produkte ist auch in diesem Fall zu dunkel. Allen zuvor geschilderten Verfahren ist zudem die Verwendung organischer Lösungsmittel gemein; unter ökologischen Gesichtspunkten sollte der Einsatz dieser Lösungsmittel jedoch weitgehend vermieden werden. Gemäß **Compt. Rend., 157, 50 (1913)** kann man auch Acetylaceton mit einem Metallhydroxid in wäßriger Lösung umsetzen. In **JP 51-54691** wird ein weiteres Verfahren zur Herstellung von Erdalkalimetallsalzen aliphatischer Ketoverbindungen beschrieben, bei dem das Erdalkalimetallhydroxid portionsweise zu dem vorgelegten aliphatischen β-Ketoester und/oder β-Diketonen in Abwesenheit eines Lösungsmittels zugegeben wird. Diese beiden Verfahren arbeiten ohne Verwendung organischer Lösungsmittel. Bei diesen Verfahren zeigte sich jedoch, daß im Laufe der Reaktion eine Phase extrem hoher Viskosität - bis zum Stillstand des Mischorgans - durchlaufen wird und daher die Handhabung des Prozesses sich schwierig gestaltet, außerdem kam es zu Anbackungen des Produktgemisches am Reaktor. Des weiteren weist das erhaltene Produkt immernoch eine relativ dunkle Farbe und eine grobe Körnung auf.

Die Aufgabe der vorliegenden Erfindung hat somit darin bestanden, ein lösungsmittelfreies Verfahren zu entwickeln, mit Hilfe dessen man hellfarbige, feinkörnige Produkte erhält und bei dem das Auftreten einer Phase extrem hoher Viskosität vermieden werden kann, um so eine leichter handhabbare Reaktionsführung zu erreichen.

### Beschreibung der Erfindung

Gegenstand der Erfindung ist demnach ein Verfahren zur Herstellung von Erdalkalimetallsalzen aliphatischer β-Ketoverbindungen durch die Umsetzung von Erdalkalimetallhydroxiden mit aliphatischen β-Ketoestern und/oder β-Diketonen in Abwesenheit eines Lösungsmittels zu den entsprechenden Erdalkalimetallsalzen, bei dem man
a) zunächst das pulverförmige Erdalkalimetallhydroxid vorlegt und die aliphatische β-Ketoverbindung portionsweise zudosiert und
b) anschließend die Reaktionsmischung einem Trocknungsschritt unterwirft.

Es wurde nun überraschenderweise gefunden, daß durch die portionsweise Zudosierung der β-Ketoverbindung zu dem pulverförmig vorgelegten Erdalkalimetallsalz das Durchlaufen einer hochviskosen Phase vermieden werden kann und dadurch die Reaktionsführung einfacher verläuft. Es kommt auch nicht zum Anbacken von Produkt am Reaktor. Des weiteren zeichnen sich die in Ausbeuten von mehr als 90 % erhaltenen Produkte durch eine helle Farbe aus und sie sind sehr feinkörnig.

### Aliphatische β-Ketoester und aliphatische β-Diketone

Die erfindungsgemäß einzusetzenden aliphatischen β-Ketoester und aliphatischen β-Diketone werden durch die folgende Formel repräsentiert

R¹COCH₂COR² (I),

worin R¹ eine Alkylgruppe und R² eine Alkyl- oder Alkoxygruppe repräsentieren. Besonders bevorzugt im Rahmen dieser Erfindung sind Verbindungen, in denen R₁ und R₂ jeweils eine kurzkettige Alkyl- oder Alkoxygruppe mit 1 bis 3 Kohlenstoffatomen repräsentieren. Insbesondere bevorzugt sind Ethylacetoacetat, Methylacetoacetat und Acetylaceton.

### Erdalkalimetallhydroxide

Erfindungsgemäß lassen sich alle Erdalkalimetallhydroxide einsetzen, bevorzugt sind jene ausgewählt aus der Gruppe die gebildet wird durch Calciumhydroxid, Magnesiumhydroxid und Bariumhydroxid. Die aliphatische β-Ketoverbindung und das Erdalkalimetallhydroxid werden vorzugsweise in stöchiometrischen Verhältnisssen eingesetzt (2:1).

### Verfahren

Das Erdalkalimetallhydroxid wird pulverförmig in einem Kneter, Trockner oder Mischer vorgelegt und anschließend die flüssige β-Ketoverbindung bei Raumtemperatur zudosiert und die beiden Edukte werden innig vermengt. Die Zudosierung erfolgt derart, daß das Reaktionsprodukt zu jeder Zeit pulverförmig bleibt, es weist dadurch eine gleichbleibende Viskosität auf. Da die Reaktion exotherm verläuft, steigt die Reaktionstemperatur im Laufe der Zeit an, sie übersteigt jedoch nicht einen Wert von 40°C. Nachdem die β-Ketoverbindung vollständig zudosiert wurde und die Hauptreaktion abgeschlossen ist, erfolgt der Trocknungsschritt. Das Reaktionswasser kann durch Anlegen eines Vakuums von 10 bis 50 mbar, vorzugsweise 20 bis 40 mbar und/oder Dampfdruckerniedrigung durch einen Gasstrom vorzugsweise eines Inertgases und/oder durch Temperaturerhöhung auf Werte zwischen 60 und 150, vorzugsweise 70 und 120 °C entfernt werden. Es werden Ausbeuten von mehr als 90 % erreicht. Nach dem Trockenvorgang fällt das Erdalkalimetallsalz als feinkörniges, pulvriges Produkt an.

In einer besonders bevorzugten Ausführungsform wird die Reaktion bei Raumtemperatur in einem List-Trockner unter inniger Vermengung der Reaktanden durchgeführt. Nach Beendigung der Zudosierung der β-Ketoverbindung zu dem pulverförmig vorgelegten Erdalkalihydroxid und dem Ende der Hauptreaktion wird zunächst mit einem Inertgas, vorzugsweise Stickstoff ein Großteil des Reaktionswassers ausgetrieben, anschließend wird der Reaktionsapparat auf eine Temperatur zwischen 90 und 120°C beheizt und bei einem Druck zwischen 30 und 40 mbar das restliche Wasser ausgetrieben, ohne, daß es zum Austritt des Acetylacetons kommt. Dadurch wird das Reaktionsgleichgewicht verschoben und die Ausbeute erhöht.

### Beispiele

### Beispiel 1

In einem 51-List-Trockner wurden 740 g Calciumhydroxid (9,98 mol) vorgelegt und 2000 g Acetylaceton (19,98 mol) innerhalb von 40 Minuten unter inniger Vermengung der Reaktanden zudosiert Die Hauptmenge an Reaktionswasser wurde gegen Ende der Reaktion durch Stickstoffspülung ausgetrieben. Anschließend wurde an das System Vakuum angelegt. Bei einem Druck von 30 mbar wurde der Apparat auf 70°C erhitzt und so die Restfeuchte ausgetrieben. Als Produkt wurde ein feines helles Pulver erhalten. Der Umsatz an Calciumhydroxid betrug 94 %.

### Vergleichsbeispiel 1

In einem List-Trockner wurden 2000 g Acetylaceton (19,98 mol) vorgelegt und 740 g Calciumhydroxid (9,98 mol) unter innigem Vermengen der Reaktanden, zudosiert. Während des Zudosierens des Calciumhydroxids kam es zur Bildung großer Verklumpungen und zum Anbacken von Substanz am Reaktionsgefäß und den Schaufeln. Das Reaktionswasser wurde durch Stickstoffspülung und anschließendes Anlegen eines Vakuums von 30 mbar bei gleichzeitiger Temperaturerhöhung auf 70 °C ausgetrieben. Das erhaltene Produkt war ein grobes Pulver mit deutlichen Verklumpungen sowie einer gelblich-dunklen Farbe. Der Umsatz an Calciumhydroxid betrug 52 %.

## Patentansprüche

1. Verfahren zur Herstellung von Erdalkalimetallsalzen aliphatischer β-Ketoverbindungen durch die Umsetzung von Erdalkalimetallhydroxiden mit aliphatischen β-Ketoestern und/oder β-Diketonen in Abwesenheit eines Lösungsmittels zu den entsprechenden Erdalkalimetallsalzen, **dadurch gekennzeichnet,** daß man
a) zunächst das pulverförmige Erdalkalimetallhydroxid vorlegt und die aliphatische β-Ketoverbindung portionsweise zudosiert und
b) anschließend die Reaktionsmischung einem Trocknungsschritt unterwirft.

2. Verfahren nach den Anspruch 1, **dadurch gekennzeichnet,** daß man als aliphatische β-Ketoester Ethylacetoacetat und/oder Methylacetoacetat einsetzt.

3. Verfahren nach den Ansprüchen 1 und 2, **dadurch gekennzeichnet,** daß man als aliphatisches β-Diketon Acetylaceton einsetzt.

4. Verfahren nach den Ansprüchen 1 bis 3, **dadurch gekennzeichnet,** daß man Erdalkalimetallhydroxide einsetzt, die ausgewählt sind aus der Gruppe, die gebildet wird von Calciumhydroxid, Magnesiumhydroxid und/oder Bariumhydroxid.

5. Verfahren nach den Ansprüchen 1 bis 4, **dadurch gekennzeichnet,** daß man die aliphatische β-Ketoverbindung bei Raumtemperatur zudosiert.

6. Verfahren nach den Ansprüchen 1 bis 5, **dadurch gekennzeichnet,** daß man die Reaktion unter innigem Vermengen der Edukte in einem Kneter, Mischer oder Trockner durchführt.

7. Verfahren nach den Ansprüchen 1 bis 6, **dadurch gekennzeichnet,** daß man die Trocknung durch Druckerniedrigung, Temperaturerhöhung und/oder Strippen mit einem Inertgas durchführt.

## Claims

1. A process for the production of alkaline earth metal salts of aliphatic β-keto compounds by reaction of alkaline earth metal hydroxides with aliphatic β-keto esters and/or β-diketones in the absence of a solvent to form the corresponding alkaline earth metal salts, characterized in that
a) the powder-form alkaline earth metal hydroxide is introduced first into the reactor and the aliphatic β-keto compound is added in portions and
b) the reaction mixture is subjected to a drying step.

2. A process as claimed in claim 1, characterized in that ethyl acetoacetate and/or methyl acetoacetate is/are used as the aliphatic β-ketoester(s).

3. A process as claimed in claims 1 and 2, characterized in that acetyl acetone is used as the aliphatic β-diketone.

4. A process as claimed in claims 1 to 3, characterized in that alkaline earth metal hydroxides selected from the group consisting of calcium hydroxide, magnesium hydroxide and/or barium hydroxide are used.

5. A process as claimed in claims 1 to 4, characterized in that the aliphatic β-keto compound is added at room temperature.

6. A process as claimed in claims 1 to 5, characterized in that the reaction is carried out in a kneader, mixer or dryer in which the educts are thoroughly mixed.

7. A process as claimed in claims 1 to 6, characterized in that the drying step is carried out by reducing the pressure, increasing the temperature and/or stripping with an inert gas.

## Revendications

1. Procédé de production de sels de métaux alcalino-terreux de composés β-cétoaliphatiques par réaction d'hydroxydes de métaux alcalino-terreux avec des β-céto esters aliphatiques et/ou des β-dicétones en l'absence d'un solvant pour donner les sels de métaux alcalino-terreux correspondants,
caractérisé en ce que
a) on dispose tout d'abord l'hydroxyde de métal alcalino-terreux pulvérulent et on dose en plusieurs fois le composé β-céto aliphatique et
b) ensuite on soumet le mélange réactionnel à une étape de séchage.

2. Procédé selon la revendication 1,
caractérisé en ce qu'
on utilise comme β-cétoester aliphatique l'acétoacétate d'éthyle et/ou l'acétoacétate de méthyle.

3. Procédé selon les revendications 1 et 2,
caractérisé en ce qu'
on utilise comme β-dicétone aliphatique l'acétylacétone.

4. Procédé selon l'une quelconque des revendications 1 à 3,
caractérisé en ce qu'
on utilise des hydroxydes de métaux alcalino-terreux qui sont choisis dans le groupe formé par l'hydroxyde de calcium, l'hydroxyde de magnésium et/ou l'hydroxyde de baryum.

5. Procédé selon l'une quelconque des revendications 1 à 4,
caractérisé en ce qu'
on dose le composé β-cétoaliphatique à la température ambiante.

6. Procédé selon l'une quelconque des revendications 1 à 5,
caractérisé en ce qu'
on conduit la réaction en mélangeant intimement les éduits dans une pétrisseuse, un mélangeur ou un dessicateur.

7. Procédé selon l'une quelconque des revendications 1 à 6,
caractérisé en ce qu'
on effectue le séchage en abaissant la pression, en élevant la température et/ou en purifiant avec un gaz inerte.
